(19) Europäisches Patentamt / European Patent Office / Office européen des brevets

(11) **EP 3 781 122 B1**

(12) **EUROPEAN PATENT SPECIFICATION**

(45) Date of publication and mention of the grant of the patent:
**06.09.2023 Bulletin 2023/36**

(21) Application number: **19717935.1**

(22) Date of filing: **18.04.2019**

(51) International Patent Classification (IPC):
**A61K 9/00** *(2006.01)*      **A61K 39/235** *(2006.01)*
**A61K 47/02** *(2006.01)*      **A61K 47/18** *(2017.01)*
**A61K 47/26** *(2006.01)*      **A61K 9/08** *(2006.01)*
**A61K 47/42** *(2017.01)*      **A61K 35/761** *(2015.01)*
**A61P 35/00** *(2006.01)*      **C12N 7/00** *(2006.01)*
**C12N 15/86** *(2006.01)*

(52) Cooperative Patent Classification (CPC):
**A61K 9/0019; A61K 9/08; A61K 47/02;**
**A61K 47/18; A61K 47/183; A61K 47/26;**
**A61K 47/42; C12N 7/00; C12N 15/86;**
C12N 2710/10343; C12N 2710/10351

(86) International application number:
**PCT/EP2019/060102**

(87) International publication number:
**WO 2019/202083 (24.10.2019 Gazette 2019/43)**

(54) **STABLE ADENOVIRUS COMPOSITIONS**

STABILE ADENOVIRUSZUSAMMENSETZUNGEN

COMPOSITIONS D'ADÉNOVIRUS STABLES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.04.2018 EP 18382269**

(43) Date of publication of application:
**24.02.2021 Bulletin 2021/08**

(73) Proprietor: **Laboratorio Reig Jofré, S.A.**
**08970 Sant Joan Despí (ES)**

(72) Inventors:
• **LAHUERTA BUIRA, Gemma**
**08970 Sant Joan Despí (ES)**
• **CARBAJAL NAVARRO, Eva Nuria**
**08970 Sant Joan Despí (ES)**
• **ROSELL I VIVES, Elisabet**
**08970 Sant Joan Despí (ES)**
• **NIKOLIC, Sasa**
**08970 Sant Joan Despí (ES)**
• **LÓPEZ ORTIZ, Lluna**
**08970 Sant Joan Despí (ES)**

(74) Representative: **ZBM Patents - Zea, Barlocci & Markvardsen**
**Rambla Catalunya, 123**
**08008 Barcelona (ES)**

(56) References cited:
**WO-A1-2017/013169      US-A1- 2006 205 080**
**US-A1- 2008 102 508      US-B2- 7 456 009**

• **ALTARAS N E ET AL: "Production and Formulation of Adenovirus Vectors", ADVANCES IN BIOCHEMICAL ENGINEERING, BIOTECHNOL, SPRINGER, BERLIN, DE, vol. 99, November 2005 (2005-11), pages 193-260, XP009105897, ISSN: 0724-6145, DOI: 10.1007/10_008 cited in the application**

Remarks:
The file contains technical information submitted after the application was filed and not included in this specification

## Description

### Technical Field

The present invention relates to the field of adenovirus formulations and to methods for preparing them.

### Background Art

In the one side it is widely known that virus are used as tools for the delivery of nucleic acids to target cells, and thus they are employed as gene-delivery vehicles for gene therapies but also as molecular biology tools in research. On the other side, some inactivated and attenuated viruses are also employed in vaccine formulations.

In any of the above-referred particular uses of the viruses, virus particles have to maintain their structure, not only to be able to infect cells but also to perform their biological activity, whatever it is. However, viral particles are damaged during the formulation processes, as well as due to moderate or high temperatures. For this reason many of the viruses for gene therapy and for molecular biology tools are formulated in buffers containing glycerol and then they are stored at -80 °C. This methodology is expensive due to the need of low temperatures for storage, thus it is difficult to be put into practice in remote zones, and its implementation is low. In addition, this methodology using glycerol has the disadvantage that glycerol is toxic for cells and it has to be depleted from the formulation before its use or, at least, the formulation with the desired amount of viruses has to be dissolved in order glycerol remains at non-toxic levels.

Another methodology widely employed for the formulation of stable virus compositions is lyophilisation. With lyophilisation an improved long-term storage stability of virus is expected, due to the water content reduction in the formulation. This methodology is employed for formulations comprising adenovirus, adeno-associated virus, retroviruses, herpes virus, rotavirus, pox viruses and vaccinia viruses. A lyophilised formulation is also helpful during manipulation of this kind of biological material in warm zones or in cases where no refrigeration is possible for a time (i.e. during vaccination campaigns in warm countries).

In general terms, stable adenovirus (and of other virus types) formulations include the viral particles in a bulking agent and one or more protectants as pharmaceutical excipients. The bulking agent is mainly a sugar or mixture of sugars with the aim of serving as the main solid in a lyophilised cake comprising adenovirus. Buffer compositions are also employed as excipients. In the particular case of lyophilised formulations the excipients contain protectants, such as cryoprotectants, and/or lyoprotectants (sometimes being the same compound). Examples of these types of protectants are non-reducing sugars, such as trehalose or sucrose. Particular lyoprotectants are albumin, amino acids, maltodextrins and surfactants, such as Tween-20. Albumin is also used as a protein to stabilize.

Example of stable virus formulations are disclosed in patent application with publication number US20080102508 (Introgen Therapeutics Inc.). In this document the long-term stability of several lyophilized adenovirus containing compositions is disclosed. The lyophilised compositions comprise mannitol (at least 3 % by weight), human serum albumin, glycerol (1% by weight), sucrose (also termed saccharose) and Tris buffer with $MgCl_2$. By determining viral particle recoveries (using HPLC) and plaque forming units (PFU) it is concluded that viruses are stable at the two assayed temperatures (-20 °C and 4 °C), and also that residual moisture after drying is an important parameter affecting storage stability. Document US20080102508 also discloses liquid stable adenovirus containing compositions. These liquid compositions comprise a mixture of excipients selected from mannitol, sucrose, glycine, arginine, urea, polyethylenglycol, all disclosed combinations in 10 mM-tris buffer with 1 % glycerol and 1 mM $MgCl_2$. Although the formulations disclosed in US20080102508, either lyophilised or liquid, are effective in terms of preserving virus structure, glycerol is also present and, thus dilution to non-toxic levels is to be done prior to their use.

Also the patent document with publication number US7456009 (Merck & Co, Inc) discloses virus liquid formulations with enhanced stability in a range of temperatures of 2-8 °C. The formulations comprise adenovirus, inhibitors of free radical oxidation, and one sugar. In some particular examples, the formulations are buffered with Tris-HCl and comprise histidine or EDTA, NaCl, $MgCl_2$ or $CaCl_2$, sucrose, polysorbate-80 and mannitol. Exemplified stable compositions after freeze and thaw cycles are Tris-buffered compositions comprising polysorbat-80, sucrose, NaCl and $MgCl_2$. Although multiple formulations are exemplified as being stable in the range of 2-8 °C at a neutral pH, and they are liquid and ready to use for example as intramuscular vaccines, few are the exemplified compositions that can be lyophilised.

Finally, another handicap in the preparation of adenovirus formulations, in particular those to be further lyophilised, is the pH value and the buffer system used to assure the pH maintenance during manipulation. On the one side, phosphate-based buffer systems tend to precipitate during lyophilisation and this damage the virus particles. It is for this reason that many adenovirus compositions are buffered with Tris-HCl, which does not precipitate. However, using a protonated buffer (Tris-HCl, for example) implies the disadvantage that during lyophilisation meanwhile the composition is being frozen some microenvironments are formed. In these microenvironments and due to the presence of protons (H+) from the buffer system, the pH is lowered and the acidic conditions damage the virus particles too.

Thus, there is still a need of providing stable virus formulations, as well as a need of formulations that can be processed both as liquid and as lyophilised formulations without the above-mentioned drawbacks.

## Summary of Invention

The inventors provide herein a stabilized adenovirus formulations or pharmaceutical adenovirus containing compositions that are usable in gene therapy or for vaccination. Advantageously, despite of the fact that the proposed formulations do not contain glycerol, they are stable in lyophilized form for a long period of time, as well as in liquid form. The liquid stable formulations allow the processing at industrial scale, as will be depicted below, without compromising adenovirus structure, mainly nucleocapsid, and so assuring the amount of effective infective units. The inventors developed optimal adenovirus formulations for lyophilisation, wherein the lyophilized adenovirus remain stable and have residual moisture from 1 % to 3 %. This residual moisture (RM) is the amount of bound water that remains in a freeze-dried product after complete cycle of lyophilisation. The Karl-Fisher Technique for testing for residual moisture determines the water content by coulometric titration. This is measured as the weight percentage of water remaining to the total weight of the dried product. RM is an indicator of stability as exposure to moisture during storage that can destabilize a product.

Thus, a first aspect of the invention is a pharmaceutical adenovirus containing composition, which is free of glycerol, comprising:

- a therapeutically effective amount of one or more adenovirus type;
- a buffer system comprising tris(hydroxymethyl) aminomethane (Tris) and glycine;
- a cryoprotectant mixture comprising (a) trehalose, (b) mannitol, and (c) saccharose;
- albumin; and
- a mixture of inorganic salts comprising sodium chloride and magnesium chloride.

Inventors have surprisingly found that with the particular combination of cryoprotectants and using as buffer also the particular combination of Tris and glycine, stable adenovirus formulations could be obtained, in such a way that no significant loss of infective units occurred in lyophilized compositions measured at different storage times. In addition, this stability property could be achieved with formulations void of glycerol. Thus, any additional step of dissolving or eliminating toxic glycerol before their use can be avoided. Particular amounts of the compounds allowed, in addition, minimizing loss of infective units during lyophilisation. Further, the combination of compounds with the buffer system comprising Tris and glycine resulted in a composition with a pH that was maintained in liquid and during lyophilisation.

As will be illustrated in the examples, the protection of virus particles took place either in liquid, but also during lyophilisation of the composition, and once lyophilised. This was mainly due because all these components in the composition embraced adenovirus particles in a behaviour that did not damage the proteins of the virion for penetrating into target cells. In addition, the presence of albumin favoured adsorption of the viral particles thereon, which in turn aimed to protect the particles from proteases. Finally, as a known lyoprotectant compound, albumin also helped in the lyophilisation process to obtain a stable cake together with the remaining solids, mainly trehalose, mannitol and saccharose.

Particularly advantageous is the buffer system comprising Tris and glycine. This aprotic buffer system effectively buffered the composition in liquid and lyophilized form, as well as during lyophilisation process, while avoiding the presence of protons ($H^+$) in the mixture. As above indicated, protons favour the lowering of the pH in microenvironments created during lyophilisation. This lowering of the pH is responsible of the damage of virus particles, mainly due to an aggregation process.

Thus, the use of an aprotic buffer system comprising Tris and glycine in a composition comprising adenovirus is also disclosed. Using such a buffer is, in particular, advantageous for formulations with adenovirus that are to be lyophilized. As will be depicted below, the maintenance of the pH of from 7 to 9 with this aprotic buffer system implied a less extent of particle aggregation, which aggregation is translated to a loss of functionality. In addition, this aprotic buffer system do also contributed, together with the other ingredients of the composition to stabilize the composition in terms of infective units.

Also disclosed is a pharmaceutical adenovirus containing composition comprising one or more adenovirus type and an aprotic buffer system comprising tris(hydroxymethyl) aminomethane and glycine.

A second aspect of the invention is a method for preparing a pharmaceutical adenovirus containing composition as above, the method comprising the following steps:

(a) preparing a solution in water by adding a cryoprotectant mixture comprising trehalose, mannitol, saccharose; a buffer system comprising tris(hydroxymethyl) aminomethane and glycine; and a mixture of inorganic salts comprising sodium chloride and magnesium chloride;
(b) sterilization filtration of the solution obtained in step (a);

(c) adding albumin under sterile conditions;

(d) maintaining the solution of step (c) under refrigeration at a temperature from 2°C to 10 °C and under agitation for the necessary period of time to obtain an homogenized solution comprising the buffer system, the inorganic salts and the cryoprotectant mixture;

(e) mixing the homogenized solution of step (d) with an effective amount of adenovirus to obtain a liquid adenovirus composition.

This method can be performed in non-expensive conditions in terms of the time required for carrying out the process, in terms of reagents employed and in terms of devices and tools required.

Pharmaceutical compositions as defined above comprising one or more adenovirus types are usable as source of effective adenovirus infective units and, thus, they can be used in many of the common uses in which adenovirus are usually employed. For example, the pharmaceutical compositions can be used as vaccines for promoting a response against the selected adenovirus type or types. Thus, another aspect of the invention is a pharmaceutical composition as defined above for use as a vaccine. Other uses of adenovirus include gene-therapy of certain cancers. Thus, in yet another aspect the invention relates to the pharmaceutical composition as defined above for use as a gene-therapy agent.

The invention also discloses buffered compositions suitable for stabilizing adenovirus in particular when these have to be stored in lyophilized form, said compositions being free of glycerol and comprising:

- a buffer system comprising tris(hydroxymethyl) aminomethane (Tris) and glycine;
- a cryoprotectant mixture comprising (a) trehalose, (b) mannitol, and (c) saccharose;
- albumin; and
- a mixture of inorganic salts comprising sodium chloride and magnesium chloride.

This buffered composition corresponds, indeed, to the same composition disclosed in the first aspect but without any adenovirus particles. The buffered composition supposes an intermediate composition prepared to receive any desired amount of adenovirus particles, and that assures preservation of these particles in liquid and lyophilized form. As above, no glycerol is used in the preservation of adenovirus and thus it has not to be removed anymore, thus opening the option of maintaining adenovirus in a mixture which is non-toxic and can directly be used as a pharmaceutical composition for mammals, including human.

Another aspect of the invention is the use of a buffered composition comprising:

- a buffer system comprising tris(hydroxymethyl) aminomethane (Tris) and glycine;
- a cryoprotectant mixture comprising (a) trehalose, (b) mannitol, and (c) saccharose;
- albumin; and
- a mixture of inorganic salts comprising sodium chloride and magnesium chloride; as an adenovirus stabilizing composition.

## Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

The expression "adenovirus formulation" or "adenovirus containing composition" are used herewith as synonymous expressions and they relate to compositions in which, besides the desired amount of viral particles there are also appropriate excipients accompanying said viruses. These compositions are in a form selected from a solid, in particular as a fine powder, lyophilized or dried; as compressed tablets; as capsules containing the fine powder; or in liquid form.

The term "stable" means that in spite of freeze and thaw cycles the adenovirus composition has an infectivity from 60 to 100 % in relation to initial infectivity and after having been lyophilized and stored at a temperature from 5 °C to -20 °C. Infectivity is determined and measured as infective units (IU), which are the number of infective viral particles, thus functional viral particles. There are many standardized protocols for the titer of the infective units in a sample. One standardized protocol is that in which the composition with the viral particles is used at different dilutions to infect cell lines prepared to express a gene in *trans* (E1 gene) needed to express adenoviral hexon. The hexon protein is required for adenoviral replication. This hexon protein is then detected in the cells by immunoassay. An antibody anti-hexon is used together with a secondary antibody conjugated to a horseradish peroxidase (HRP) and detected by colorimetry when HRP reacts with diaminobenzidine (DAB). A dark precipitate is produced only in the cells expressing hexon protein. These dark cells are then counted under a standard laboratory microscope to calculate viral titer by extrapolating to a standard curve. The formula to obtain the IU/ml is as follows:

(Average number of positive cells/fiel x field/well) / (Volume of diluted virus used per well (ml) + dilution factor). An example of a kit for performing such a protocol is the AdEasy Viral Titer Kit (Agilent Technologies).

The expression "therapeutically effective amount" as used herein, refers to the amount of a compound that, when administered, is sufficient to prevent development of, or alleviate to some extent, one or more of the symptoms of the disease which is addressed. The particular dose of compound administered according to this invention will of course be determined by the particular circumstances surrounding the case, including the compound administered, the route of administration, the particular condition being treated, and the similar considerations.

The expression "pharmaceutically acceptable excipients or carriers" refers to pharmaceutically acceptable materials, compositions or vehicles. Each component must be pharmaceutically acceptable in the sense of being compatible with the other ingredients of the pharmaceutical composition. It must also be suitable for use in contact with the tissue or organ of humans and animals without excessive toxicity, irritation, allergic response, immunogenicity or other problems or complications commensurate with a reasonable benefit/risk ratio.

A "cryoprotectant" is a substance used to protect biological tissue, such as adenovirus particles, from freezing damage (i.e. that due to ice formation). Cryoprotectants operate by increasing the solute concentration in cells. However, in order to be biologically viable they must easily penetrate and must not be toxic to cells. Similar to cryoprotectants, some molecules protect freeze-dried material, and they are called "lyoprotectants", these molecules are typically polyhydroxy compounds such as sugars (mono-, di-, and polysaccharides), polyalcohols, and their derivatives. Trehalose and sucrose are natural lyoprotectants. Some molecules are both labelled as cryoprotectants and as lyoprotectants. Other known lyoprotectants include albumin (i.e. human serum albumin), polyethylene glycol, glycine, proline, lysine, alanine, some surfactants such as polysorbate-20 or -80, maltodextrins and certain starches. When in the present invention, trehalose, saccharose, and mannitol are mentioned they include any dehydrated or hydrated forms of the same. In the same way, there are included any pharmaceutically acceptable salts and esters. Examples of hydrated forms are, in particular trehalose dihydrate. Being the compounds in the cryoprotectant mixture of the invention sugars with several alcohol (-OH) groups, esters of them with non-toxic organic acids can also be used. Examples of organic acids are carboxylic acids of formula $CO_2$-$(C_1$-$C_{19})$-alkyl or $CO_2(C_1$-$C_{19})$-alkenyl, thus defining a $(C_1$-$C_{20})$-alkanoyl or alkenoyl substituents esterifying one or more of the -OH groups in the sugars. In particular, the sugars are esterified with fatty acids selected from the group consisting of lauryl acid, myristic acid, palmitic acid, steraric acid, and oleic acid.

The term "salt" used herewith relates to "pharmaceutically acceptable salts" formed from pharmaceutically acceptable non-toxic acids including inorganic or organic acids. There is no limitation regarding the salts, except that if used for therapeutic purposes, they must be pharmaceutically acceptable. In addition, the concept of "salt" encompasses any of the hydrated or the dehydrated forms of the same. In the particular case of the magnesium dichloride it encompasses magnesium dichloride hexahydrate.

The term "vaccine" as used herein, relates to an immunogenic composition accompanied by adequate excipients and/or carriers, that when administered to an animal, elicits, or is able to elicit, directly or indirectly, an immune response in the animal. Particularly, the vaccines of the present invention elicit an immunological response in the host of a cellular or antibody-mediated type upon administration to the subject that it is protective. The term "immunogenic" or "immunological composition" refers to material which elicits an immunological response in the host of a cellular or antibody-mediated immune response type to the composition upon administration to a vertebrate, including humans.

As "gene-therapy agent" is to be understood as a product, in particular a virus to perform a gene therapy, which is disclosed as the therapeutic delivery of a nucleic acid into a patient's cells as a drug to treat a disease. Cancers are being faced nowadays by gene-therapy using adenovirus as vectors.

For the purposes of the invention, any ranges given include both the lower and the upper end-points of the range. Ranges given, such as temperatures, times, sizes, and the like, should be considered approximate, unless specifically stated.

As indicated, the invention relates to new pharmaceutical adenovirus containing compositions, which are highly stable in terms of preserving virus infectivity, either in liquid, as well as in lyophilized form, being said infectivity measured as infective units per ml (as indicated above). These compositions comprise as pharmaceutically acceptable excipients:

- a buffer system comprising tris(hydroxymethyl) aminomethane (Tris) and glycine;
- a cryoprotectant mixture comprising (a) trehalose, (b) mannitol, and (c) saccharose;
- albumin; and
- a mixture of inorganic salts comprising sodium chloride and magnesium chloride.

In a particular embodiment, the pharmaceutical composition comprises additional pharmaceutically acceptable excipients and/or carriers. In particular those other excipients and/or carriers that make the composition suitable to be used in an injectable composition, such as particular solvents, more in particular, water. Other additional excipients and/or carriers are compounds suitable for making the compositions orally administered.

In the same way, the pharmaceutical compositions of the first aspect comprise, in another particular embodiment, additional therapeutically effective amounts of other ingredients, such as adjuvants commonly used in vaccination.

Inventors determined that a simplified free of glycerol formulation was able to preserve adenovirus particles integrity. This simplified formulations included as buffer system only Tris and glycine. Thus, in a particular embodiment of the pharmaceutical composition according to the first aspect, Tris and glycine are the only buffer components in the composition.

In a particular embodiment, the pharmaceutical composition consists of:

(a) a therapeutically effective amount of one or more adenovirus type;
(b) Tris and glycine;
(c) trehalose;
(d) mannitol;
(e) saccharose;
(f) albumin;
(g) sodium chloride; and
(h) magnesium chloride.

In another particular embodiment, the pharmaceutical composition consists of:

(a) a therapeutically effective amount of one or more adenovirus type;
(b) Tris and glycine;
(c) trehalose;
(d) mannitol;
(e) saccharose;
(f) albumin;
(g) sodium chloride;
(h) magnesium chloride; and
(i) additional pharmaceutically acceptable excipients and/or carriers.

In even a more particular embodiment, the additional pharmaceutically acceptable excipient and/or carrier is water. More in particular water for injectables.

Further, in another particular embodiment of the pharmaceutical composition, optionally in combination with any embodiment above or below, in the cryoprotectant mixture the weight ratio of trehalose, mannitol and saccharose is from 6.5:1.5:0.5 to 7.5:2.5:1.5. More in particular it is 7:2:1.

As will be depicted in the examples below, inventors have proved that different final amounts of these three cryoprotectant components but within this weight ratio in an adenovirus containing composition, allow maintaining infective units after lyophilisation and for several months. In addition, this infective capacity is maintained when the lyophilised compositions are stored at different temperatures ranging from -20 °C to 25 °C.

In another particular embodiment, the pharmaceutical composition is a liquid aqueous composition at a temperature from 14 °C to 35 °C.

Liquid compositions according to the invention imply the advantage that they are stable for a period of time that can be processed at industrial scale as a previous composition that will be further lyophilized. In particular, it has been observed that they are stable, which means that no significant loss of infectivity is observed, which means that infective units/ml are maintained at least during 8-12 hours. Stability data of a liquid composition according to the invention are depicted below in the examples.

Typical adenovirus stabilizing compositions mainly comprise a buffer system, sugars as bulking agents and as cryoprotectants, and salts of divalent cations. The buffer system is important not only to provide the adequate pH for viral particles, but also to ensure that this pH will be maintained in the multiple several conditions the particles will encounter. So that, it is known that the buffer system has to work also during freezing processes without losing their effects and thus damaging viral structure. In another particular embodiment of this liquid aqueous composition the pH is from 7.0 to 9.0, the concentration of Tris is from 5 to 30 mM, and the concentration of glycine, is from 0.15 M to 0.25 M. Glycine is thus used in the pharmaceutical composition of the invention in the particular amounts that make it an ingredient of the buffer system more than acting as a bulking agent.

In a more particular embodiment the concentration of tris(hydroxymethyl) aminomethane is from 15 to 25 mM, more in particular is 25 mM. In another particular embodiment, the concentration of glycine is from 0.15 M to 0.20 M, more in particular is 0.19 M. In even a more particular embodiment, the concentration of Tris is 25 mM, the concentration of glycine is 0.192 M and the final pH is from 8.0 to 9.0.

In another particular embodiment of the liquid aqueous pharmaceutical compositions the weight/volume percentage of trehalose is from 7 % to 15 %, more in particular is from 7 % to 11 %, and even more in particular is of 10.5%;

the weight/volume percentage of mannitol is from 2 % to 3, in particular is 3 %;
the weight/volume percentage of saccharose is from 1 % to 2 %, in particular from 1 % to 1.5 %, more in particular 1.5 %; and
the weight/volume percentage of albumin is from 0.04 % to 0.5 % and even more in particular is 0.4 %;
being the weight/volume percentage of any compound defined by the amount in grams of the compound in a 100 ml of the liquid aqueous composition.

Particular weight/volume percentages of any of the compounds in combination with those weight/volume percentages of the rest of the compounds are to be considered as specifically disclosed in this description.

In another particular example, the weight/volume percentage of trehalose is of 10.5 %; the weight/volume percentage of mannitol is of 3 %; the weight/volume percentage of saccharose is of 1.5 %; and the weight/volume percentage of albumin is of 0.4 %.

In another alternative particular embodiment, the weight/volume percentage of trehalose is of 7.0 %; the weight/volume percentage of mannitol is of 2.0 %; the weight/volume percentage of saccharose is of 1.0 %; and the weight/volume percentage of albumin is of 0.4 %.

In another alternative particular embodiment, the weight/volume percentage of trehalose is of 7.0 %; the weight/volume percentage of mannitol is of 2.0 %; the weight/volume percentage of saccharose is of 1.0 %; and the weight/volume percentage of albumin is of 0.04 %. This composition with lower amounts of cryoprotectants is still able to protect the virus particles from degradation (measured as infective units) once the composition has been lyophilized and tested for infective units at several times

Compositions with a higher amounts of cryoprotectants than the previous one but still maintaining the particular weight ratio from 6.5:1.5:0.5 to 7.5:2.5:1.5, more in particular of 7:2:1 of trehalose:mannitol:saccharose are especially useful for protecting the virus particles from degradation (measured as infective units).

As will be illustrated in the examples below, other more complex compositions, used as comparative examples and with a different buffer system implied a higher loss of infective units during the lyophilisation process.

In another particular embodiment the concentration of sodium chloride is from 25 mM to 250 mM, more in particular it is from 50 mM to 100 mM, even more in particular from 60 mM to 80 mM, and even yet more in particular is from 75 mM to 80 mM. In still a more particular embodiment is 75 mM.

In another particular embodiment the concentration of magnesium dichloride is from 0.1 mM to 5.0 mM, more in particular from 0.5 mM to 3.0 mM, even more in particular from 0.5 to 2.0 mM. More in particular is from 1.0 mM to 2.0 mM. In particular is 1.0 mM.

In another particular embodiment, the adenovirus containing composition is in form of a lyophilised composition. As will be illustrated in the examples below, lyophilised compositions are stable at least for 6 months, which as above indicated means that the infective units are not loss due to disintegration of virus and/or nucleic acid (DNA) damage.

The lyophilised compositions have residual moisture from 1.0 % to 3.0 % by weight in relation to the total weight of the lyophilised cake.

Adenoviruses represent the largest non-enveloped viruses (which means without an outer lipid bilayer). Adenoviruses (members of the family Adenoviridae) are medium-sized (90-100 nm) viruses with an icosahedral nucleocapsid containing a double stranded DNA genome. They are able to be transported through the endosome (i.e., envelope fusion is not necessary). The virion also has a unique "spike" or fiber associated with each penton base of the capsid (that aids in attachment to the host cell via the receptor on the surface of the host cell. They have a broad range of vertebrate hosts; in humans, more than 50 distinct adenoviral serotypes have been found to cause a wide range of illnesses, from mild respiratory infections in young children (known as the common cold) to life-threatening multi-organ disease in people with a weakened immune system. There are 57 accepted human adenovirus types (HAdV-1 to 57) in seven species (Human adenovirus A to G).

The pharmaceutical composition according to the first aspect comprises one or more of a therapeutically effective amount of any of the adenovirus types. In particular, those hosting vertebrates, more in particular mammals, and even more in particular hosting human cells.

In a particular embodiment of the pharmaceutical composition of the invention, the one or more adenovirus type is selected from the group consisting of one or more human adenovirus of C serotype, one or more human adenovirus of D serotype, and combinations thereof.

In another more particular embodiment, the one or more adenovirus type is a human adenovirus of C serotype selected from the group consisting of Ad1, Ad2, Ad5, Ad6, and combinations thereof. Even more in particular is Ad5.

The therapeutically effective amount of the adenovirus in the pharmaceutical composition can be in form of a purified adenovirus or as an extract or lysate of cells infected with the adenovirus. Purified adenoviruses are mainly obtained by cell lysis and further separation of the virus particles in cessium chloride gradient centrifugation.

More in particular, the pharmaceutical composition comprises a therapeutically effective amount of only one adenovirus type.

In another particular embodiment, the amount of adenovirus in the pharmaceutical composition of the invention is from $1 \times 10^{10}$ vp/mL to $1 \times 10^{13}$ vp/mL. More in particular from $1 \times 10^{11}$ vp/mL to $1 \times 10^{12}$ vp/mL. "vp/mL" is the abbreviation of virus particles per millilitre of the pharmaceutical composition, and it is calculated as disclosed. Briefly, adenovirus particles concentracion is spectrophotometrically determined using the property of proteins to absorb at 277 nm, and that of double stranded DNA with a maximal of absorbance at 260 nm . Optical density detected at 260 nm (OD260) in the presence of an ionic detergent, such as SDS, is thus proportional to adenovirus particle concentration. Adenovirus particle concentration of each sample is calculated from a tripiclate analysis and considering the dilution factor and the equivalence of a value of OD260 of 1.00 being a concentration of $1.1 \times 10^{12}$ particles/ml (pv/mL).

Another aspect of the invention is a method for preparing a pharmaceutical adenovirus containing composition as disclosed above. The method comprises (a) preparing a solution in water with the cryoprotectant mixture void of albumin, the buffer system and the mixture of the inorganic salts;

> (b) sterilization filtration of the solution obtained in step (a);
> (c) adding albumin under sterile conditions;
> (d) maintaining the solution of step (c) under refrigeration at a temperature from 2°C to 10 °C and under agitation for the necessary period of time to obtain an homogenized solution comprising the buffer system, the inorganic salts and the cryoprotectant mixture; and
> (e) mixing the homogenized solution of step (d) with an effective amount of adenovirus to obtain a liquid adenovirus formulation.

The pharmaceutical adenovirus containing composition, which is free of glycerol, and comprising:

- a therapeutically effective amount of one or more adenovirus type;
- a buffer system comprising tris(hydroxymethyl) aminomethane and glycine;
- a cryoprotectant mixture comprising trehalose, mannitol, and saccharose;
- albumin; and
- a mixture of inorganic salts comprising sodium chloride and magnesium chloride;

may also be defined by its preparation process, as the composition as defined above obtainable by a method comprising the following steps:

> (a) preparing a solution in water by adding a cryoprotectant mixture comprising trehalose, mannitol, saccharose; a buffer system comprising tris(hydroxymethyl) aminomethane and glycine; and a mixture of inorganic salts comprising sodium chloride and magnesium chloride;
> (b) filtering under sterile conditions the solution of step (a) through a mesh with a pore diameter equal or lower than 0.22 $\mu$m:
> (c) adding albumin under sterile conditions;
> (d) maintaining the solution of step (c) under refrigeration at a temperature from 2°C to 10 °C and under agitation for the necessary period of time to obtain an homogenized solution comprising the buffer system, the inorganic salts and the cryoprotectant mixture;
> (e) mixing the homogenized solution of step (d) with an effective amount of adenovirus to obtain a liquid pharmaceutical adenovirus containing composition.

In a particular embodiment of the method, step (b) of sterilization filtration is performed by filtering under sterile conditions the solution of step (a) through a mesh with a pore diameter equal or lower than 0.22 $\mu$m.

Necessary period of time to obtain a homogenized solution in step (d) will be determined by the skilled man depending on the volume of manufactured batch.

In another particular embodiment of the method, it further comprises a step of lyophilisation to obtain a lyophilised pharmaceutical adenovirus containing composition, which is highly stable in terms of infective units, and which has an appropriate cake consistency.

Many lyophilisation patterns can be used, but in a particular embodiment of the invention, lyophilisation is carried out by:

- lowering the liquid pharmaceutical adenovirus containing composition to a temperature from -60°C to -50 °C, in particular for a period of time from 4 to 10 hours;
- a first drying step at a temperature from -45 °C to -30 °C at a pressure from 5.0 Pa to 8.0 Pa, in particular for a period of time from 30 to 60 hours; and
- a second drying step in which the temperature is elevated to a temperature from 10 °C to 40 °C, and which temperature is maintained for a necessary period of time to obtain a lyophilized cake with a residual humidity (also known as residual moisture) of from 1.0 % to 3.0 % by weight in the final lyophilised composition.

Residual moisture is the amount of bound water that remains in the lyophilised product. It is measured by a Karl-Fisher type coulometer (Mettler DL37, KF coulometer, Titrator 852). KFC Mode was as follows: End point 50 mV; I(pol) 10 μA; Automatic drift correction; Relative stop drift of 10 μg/min and generator without diaphragm 400 mA. The method was designed following manufacturer instructions with the solvent Hydranal Coulomat AG (Honeywell): formamida (Fluka) (70:30 v/v). Calculation of water percentage in the lyophilised sample (vial) in relation to the total weight of the lyophilised tablet (% w/w) was performed as recommended by manufacturer. Triplicates were performed for each testes sample.

The pharmaceutical composition of the invention is for use as a vaccine. Thus, present invention also relates to vaccines comprising the pharmaceutical composition of the invention. These vaccines comprise, in a particular embodiment, one or more adjuvants, which in combination with the antigenic portion of the vaccine defined by one or more adenovirus type, result in the immunogenic composition duly accompanied by the excipients, namely the buffer system, the inorganic salts, the cryoprotectant mixture, albumin and optionally additional excipients and/or carriers.

If the pharmaceutical composition of the invention is in lyophilised form, the vaccines comprising it can be orally administered as tablets, re-suspended in an appropriate solvent (i.e. water) for oral administration of for injection if this later is the preferred administration route.

Being the adenovirus good recombinant vectors, they have been also widely used in gene-therapy. Thus, the pharmaceutical adenovirus containing composition is also for use as a gene-therapy agent. This can be reformulated as a pharmaceutical composition as defined above for use in the gene-therapy of diseases and/or disorders in which either the silencing or activation or repression of certain genes takes place. Examples of these disorders are mainly several cancers. The invention thus also relates to the use of the pharmaceutical adenovirus containing composition for the preparation of a medicament for gene therapy, in particular for gene-therapy of cancer. This means that disclosed herein is the pharmaceutical composition for use in a method of treatment of a subject suffering from a diseases and/or disorder, in which either the silencing or activation or repression of certain genes takes place, by administering to the subject a therapeutically effective amount of the pharmaceutical composition of the invention.

Due to their stabilizing properties the formulations can be used as a source of stable adenovirus usually employed as vectors for molecular biology research, for example in processes of transforming cells, including mammal cells, and in particular human cells.

As above indicated the invention also discloses as intermediate product a buffered composition free of glycerol and comprising:

- a buffer system comprising tris(hydroxymethyl) aminomethane (Tris) and glycine;
- a cryoprotectant mixture comprising (a) trehalose, (b) mannitol, and (c) saccharose;
- albumin; and
- a mixture of inorganic salts comprising sodium chloride and magnesium chloride.

This buffered composition is in a particular embodiment a liquid composition. In another particular embodiment it is in form of a lyophilised composition (for example in form of fine powder or as compressed tablets). Liquid buffered compositions are ready to be mixed with the desired titre of virus particles, while lyophilized forms have first to be re-suspended in a predetermined volume of a solvent, in particular water, to further add the desired titre of virus particles.

It is also disclosed, but not part of the invention, a liquid pharmaceutical adenovirus containing composition, which is free of glycerol, comprising:

- a therapeutically effective amount of one or more adenovirus type;
- a buffer system comprising Tris-HCl in a concentration from 20 mM to 25 mM, in particular 25 mM;
- a cryoprotectant mixture comprising (a) trehalose, either a salt or ester thereof with a weight/volum percentage from 8.0 % to 12 %, in particular of 10.5 %; (b) mannitol, either a salt or ester thereof, with a weight/volume percentage from 2.0 % to 3.0 %, in particular 3 %; (c) saccharose, either a salt or ester thereof, with a weight/volum percentage from 1.0 % to 2.0 %, in particular of 1.5 %;
- albumin with a weigh/volume percentage from 0.3 % to 0.5 %, in particular 0.4 %; being the weight/volume percentage of any compound of the cryoprotectant mixture and of albumin defined by the amount in grams of the compound in a 100 ml of the total liquid aqueous composition; and

- a mixture of inorganic salts comprising sodium chloride and magnesium chloride, wherein the concentration of sodium chloride is from 25 mM to 250 mM, more in particular from 50 mM to 100 mM, more in particular from 60 mM to 75 mM, and more in particular 75 mM; and the concentration of magnesium chloride is from 0.1 mM to 5.0 mM, more in particular from 0.5 mM to 3.0 mM, more in particular from 0.5 mM to 1.5 mM, and even more in particular is 1mM.

It is also disclosed, but not part of the invention a lyophilised pharmaceutical composition obtainable by lyophilising the liquid pharmaceutical composition defined in the anterior paragraph and comprising as buffer system the Tris-HCl in a concentration from 20 mM to 25 mM, in particular 25 mM. These type of lyophilised compositions are also stable in terms of infective units when stored at -20 °C and 5 °C (See Table 2, batch LP0243_86). This lyophilised composition with a buffer system comprising Tris-HCl has a residual moisture from 1.0 % to 3.0 % by weight in the final lyophilised composition.

As indicated, it is disclosed the use of an aprotic buffer system comprising tris(hydroxymethyl) aminomethane (Tris) and glycine as buffer system in an adenovirus-containing composition. This aspect results of the observation of the inventors, according to which a buffer system comprising a mixture of Tris and glycine allowed the maintenance of pH and also the integrity of viral particles during lyophilisation. Particles also maintained their functionality (in terms of UI/ml). All this allowed that a high initial concentration of viral particles could be used in the process of manufacturing of liquid compositions comprising adenovirus. This concentration in liquid composition was not altered due to the lyophilisation process.

In a particular embodiment of the use of an aprotic buffer system comprising Tris and glycine as buffer system in an adenovirus-containing composition, said buffer system consists in the mixture of Tris and glycine. In another particular embodiment, optionally in combination with any embodiment above or below, the concentration of Tris is from 5 to 30 mM, and the concentration of glycine, is from 0.15 M to 0.25 M. In even a more particular embodiment, the concentration of Tris is from 15 to 25 mM, more in particular is 25 mM. In another particular embodiment, the concentration of glycine is from 0.15 M to 0.20 M, more in particular is 0.19 M. In even a more particular embodiment, the concentration of Tris is 25 mM, the concentration of glycine is 0.192 M and the final pH is in the adenovirus-containing composition provided by the use of the aprotic buffer system is from 8.0 to 9.0.

It is thus disclosed, pharmaceutical adenovirus containing compositions comprising one or more adenovirus type and an aprotic buffer system comprising Tris and glycine, wherein the Tris and glycine are the only buffer components in the adenovirus-containing composition.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention.

**Example**

**Stable pharmaceutical adenovirus containing compositions**

Herewith are disclosed different pharmaceutical compositions containing adenovirus. Stability of the adenovirus was assessed by determining infective units lost due to the lyophilisation step. Infective units were also assayed at several times after lyophilisation and at several storage temperatures.

Materials and methods

Employed ingredients for the preparation of different compositions were Trehalose dihydrate (Pfanstiehl), mannitol (Pfanstiehl), saccharose (Merck), Tris (Sigma Aldrich), $MgCl_2$ hexahydrate (Merck), NaCl (Merck), HCl (Sigma Aldrich), glycine (Sigma Aldrich), histidine (Merck), EDTA (Merck), Polysorbate-80 (PS-80 of Croda) and human serum albumin ( HSA of Grifols).

All the components were mixed in water at the amounts indicated in Table 1 below. The mixture of all excipients was filtered using a Millex-GV of polyvinylpyrrolidone (PVP) of 0.22 $\mu$m under laminar flow. The solution was then transferred to sterile Falcon tubes (50 ml) having low protein retention. In case the cryoprotectant mixture comprised albumin (HSA), this was added after the filtration and under laminar flow. The tubes were maintained in orbital agitation for 15 minutes and refrigerated (4-5 °C) to assure complete homogenization of all the ingredients.

All batches were formulated with equivalent Ad5 adenoviral particles of $1\times10^{12}$ vp/mL. This amount of Ad5 provided a desired adenovirus concentration in the final product at the same time high initial amounts help to stabilize the particles due to a higher protein concentration [see Altaras et al., "Production and Formulation of Adenovirus Vectors", Adv. Biochem. Engin/Biotechnol-2005, vol. no. 99, pp.: 193-260]. Viral stocks were first thawed at room temperature (14-26 °C). If cryovials were used, they were thawed and centrifuged with a pulse in order to recover the whole contents in a Falcon tube (of 15 ml). The tubes were maintained in orbital agitation for 10 minutes and refrigerated (4-5 °C). If the virus stock was already in a Falcon tube, they were directly maintained in orbital agitation for 10 minutes and refrigerated (4-5 °C). Once the virus stock was homogenized, a desired amount was transferred to Falcon of 50 ml and it was added a volume of the mixture of excipients (buffer system, cryoprotectants and inorganic salts) in order to have a final Ad5 concentration of $1 \times 10^{12}$ vp/mL. The adenovirus containing composition was then agitated under refrigeration (4-5 °C) for 10 min. Adenovirus particle concentration (vp/ml) used was in the range of the therapeutically effective. Therefore, the compositions of the invention were assayed with the proper viral titer for use in treatment.

All batches were dosed and lyophilized as follows: 0.5 ml were dosed in borosilicate type I plus vials. The lyophilisation equipment employed was Lyobeta-20 of Telstar. Vials were initially deposited on plates previously refrigerated (5-10 °C) and temperature was lowered at -5 °C for 15 minutes to assure simultaneous and homogeneous freezing of all vials. The lyophilisation pattern included a freeze cycle at -55 °C for 6 hours; a primary drying at -40 °C for 50 hours at a pressure of 6 Pa (0.06mbar); and a secondary drying with an increasing temperature gradient to finally reach 30 °C in10 hours which 30 °C were maintained for 4 hours. After the lyophilisation the vials were covered with stoppers under vacuum. Aluminium foil caps were added to the vials and they were distributed to the different storage conditions assayed.

Results

1. Effect of lyophilisation and stability data at different conditions

Next Table 1 shows the composition of several manufactured batches of the pharmaceutical composition of the invention, which initially contained an Ad5 concentration of $1 \times 10^{12}$ vp/mL. Although not indicated in the Table 1, all batches included NaCl 75 mM and $MgCl_2$ hexahydrate 1 mM. Trehalose was in the dihydrate form.

Table 1 includes also some comparative examples (batch L0243_86 and L0243_41).

For each of the batches there is indicated the infective units per millilitre (IU/ml) when Ad5 was added to the mixture of excipients, after lyophilisation and the loss during lyophilisation. IU/ml were determined applying the protocol disclosed as in the kit AdEasy Viral Titer Kit (Agilent Technologies). Briefly, using a primary monoclonal anti-hexon antibody and a secondary horseradish peroxidase conjugated anti-mouse antibody and an appropriated substrate.

Table 1. Lyophilisation process

| Batch | Composition | IU/ml initial | IU/ml after lyophilisation (t=0) | Loss during lyophilisation (%)[1] |
|---|---|---|---|---|
| LP0243_86 (comparative) | Tris-HCl 25 mM pH8.5 + trehalose 10.5 % (w/v) + mannitol 3 % (w/v) + Saccharose 1.5 % (w/v) + HSA 0.4 % (w/v) | $9.57 \times 10^{10}$ | $3.35 \times 10^{10}$ | 65 |
| LP0243_98 | Tris 25 mM Glycine 0.192 M pH8.5 + trehalose 10.5 % (w/v) + mannitol 3 % (w/v) + Saccharose 1.5 % (w/v) + HSA 0.4 % (w/v) | $1.16 \times 10^{10}$ | $7.14 \times 10^{9}$ | 38 |
| LP0243_102 | Tris 25 mM Glycine 0.192 M pH8.5 + trehalose 10.5 % (w/v) + mannitol 3 % (w/v) + | $2.54 \times 10^{10}$ | $2.22 \times 10^{10}$ | 13 |
| | Saccharose 1.5 % (w/v) + HSA 0.4 % (w/v) | | | |
| LP0243_103 | Tris 25 mM Glycine 0.192 M pH8.5 + trehalose 10.5 % (w/v) + mannitol 3 % (w/v) + Saccharose 1.5 % (w/v) + HSA 0.4 % (w/v) | $2.18 \times 10^{10}$ | $2.21 \times 10^{10}$ | 0 |
| LP0243_81 | Tris 25 mM Glycine 0.192 M pH8.5 + trehalose 7.0 % (w/v) + mannitol 2.0 % (w/v) + Saccharose 1.0 % (w/v) + HSA 0.4 % (w/v) | $2.37 \times 10^{10}$ | | |
| LP0243_26 | Tris 25 mM Glycine 0.192 M pH8.5 + trehalose 7.0 % (w/v) + mannitol 2.0 % (w/v) + Saccharose 1.0 % (w/v) + HSA 0.04 % (w/v) | $1.24 \times 10^{9}$ | $2.83 \times 10^{8}$ | 77 |

(continued)

| Batch | Composition | IU/ml initial | IU/ml after lyophilisation (t=0) | Loss during lyophilisation (%)[1] |
|---|---|---|---|---|
| LP0243_27 | Tris 25 mM Glycine 0.192 M pH8.5 + trehalose 7.0 % (w/v) + mannitol 2.0 % (w/v) + | $1.24 \times 10^9$ | $3.00 \times 10^8$ | 76 |
| | Saccharose 1.0 % (w/v) + 0.02% PS-80 + 100 $\mu$M EDTA + 10 mM Histidine+ HSA 0.04 % (w/v) | | | |
| LP0243_41 (comparative) | Tris-HCl 10 mM pH 7.5 + trehalose 7.0 % (w/v) + mannitol 2.0 % (w/v) + Saccharose 1.0 % (w/v) + 0.02% PS-80 + 100 $\mu$M EDTA + 10 mM Histidine+ HSA 0.1 % (w/v) | $3.55 \times 10^8$ | $3.35 \times 10^6$ | 99 |
| [1] Loss during lyophilisation calculated as: Loss = 100 - (Functionality at t(0) $\times$ 100/ functionality of the solution) | | | | |

Table 2 shows the stability data after storage at different conditions and for different times after lyophilisation (1 month, 3 months and 6 months).

Table 2. Stability data during storage

| Batch | Stab. cond | IU/ml t=1month | Loss[2] | IU/ml t=3months | Loss[2] | IU/ml t= 6months | Loss[2] |
|---|---|---|---|---|---|---|---|
| LP0243_86 (comparative) | -20 °C | $2,82 \times 10^{10}$ | 16 | $2,92 \times 10^{10}$ | 13 | $3,37 \times 10^{10}$ | 0 |
| | 5 °C | $2,72 \times 10^{10}$ | 19 | $2,89 \times 10^{10}$ | 14 | $3,26 \times 10^{10}$ | 3 |
| | 25 °C | $6,12 \times 10^9$ | 82 | $1,80 \times 10^9$ | 95 | $1,54 \times 10^9$ | 95 |
| LP0243_98 | -20 °C | $7,49 \times 10^9$ | 0 | $8,03 \times 10^9$ | 0 | | |
| | 5 °C | $7,22 \times 10^9$ | 0 | $7,64 \times 10^9$ | 0 | | |
| | 25 °C | $4,64 \times 10^9$ | 35 | $4,28 \times 10^9$ | 40 | | |
| LP0243_102 | -20 °C | - | - | $1,94 \times 10^{10}$ | 13 | $1,88 \times 10^{10}$ | 15 |
| | 5 °C | - | - | $1,91 \times 10^{10}$ | 14 | $1,85 \times 10^{10}$ | 17 |
| | 25 °C | - | - | - | - | $8,38 \times 10^8$ | 96 |
| LP0243_103 | -20 °C | - | - | $2,27 \times 10^{10}$ | 0 | | |
| | 5 °C | - | - | $2,12 \times 10^{10}$ | 4 | | |
| | 25 °C | $1,80 \times 10^{10}$ | 19 | $1,60 \times 10^{10}$ | 28 | | |
| LP0243_81 | -20 °C | - | - | $6,56 \times 10^9$ | - | $8,23 \times 10^9$ | - |
| | 5 °C | | - | - | - | - | - |
| | 25 °C | - | - | - | - | - | - |
| LP0243_26 | -20 °C | - | - | | | | |
| | 5 °C | - | - | | | | |
| | 25 °C | $3,10 \times 10^8$ | 0 | | | | |
| LP0243_27 | -20 °C | - | - | | | | |
| | 5 °C | - | - | | | | |
| | 25 °C | $2,13 \times 10^8$ | 29 | | | | |
| [2] Loss in stability storage assay calculated as: Loss = 100 - (Functionality of lyophilised composition at t(months) of stability $\times$ 100 / functionality of lyophilised composition at t(0)) - means not analysed. | | | | | | | |

As can be deduced from Tables 1 and 2 those pharmaceutical compositions comprising a buffer system comprising tris(hydroxymethyl) aminomethane and glycine, showed high stability in terms that after lyophilization the loss of infective units at the three tested storage temperatures was negligible or very low in comparison with the comparative example with Tris-HCl as buffer system. Interestingly, this behaviour was also observed at 25 °C, which make the pharmaceutical compositions of the invention storable in non-refrigerated conditions. Non-refrigerated conditions are the ones usually occurring during vaccination campaigns in developing countries or in cases of epidemiological emergency, when some pharmaceutical compositions have to be transported with poor conditions in remote areas. Therefore, with therapeutically effective amounts of adenovirus particles the compositions were stable for a long period of time.

In addition, those pharmaceutical compositions of the invention in which a higher loss of infective units was observed due to the lyophilisation process (batches LP0234_26 and LP0243_27), were in any case stable after storage at the tested temperature (-20 °C) as can be deduced from the loss values of 0.72 and 1.21, respectively.

On the other hand, comparative example LP0243_41 (Table 1) presented high loss of infective units during the lyophilization process and it was a more complex formulation. Comparative example LP0243_86 showed a slightly loss of infective units during lyophilisation, but a good stability when stored at -20°C and 5 °C. However, this Tris-HCl formulation was not stable at 25 °C in terms that the loss of infective units was pretty higher than the loss of the compositions of the invention at the same storing temperature. Finally, there are depicted below the infective units per ml of the batch LP0243_102 in liquid form and along 8 hours. These data aim disclosing the stability of the adenovirus compositions of the invention in liquid form.

Data are depicted in Table 3, wherein IU/ml were determined at several times (t= 0, 2, 4, and 8 hours). The test was carried out with frozen samples taken at the above indicated sample times and analysed the day after. Table 3 shows the value of a dilution of 2.5 dilution of an initial physical titer of $2.5 \times 10^{12}$ vp/ml, and an initial value of functional titer of $6.34 \times 10^{10}$ IU/ml. Again AdEasy Viral Titer Kit (Agilent Technologies) was used. Viral particles per ml (vp/ml) measured according to protocol disclosed above (equivalence of a value of OD260 of 1.00 being a concentration of $1.1 \times 10^{12}$ particles/ml (pv/mL).) are also indicated.

Table 3. Loss of virus particles during liquid processing

| LP0243_102 | Time (h) | vp/ml | IU/ml |
|---|---|---|---|
| | | [*]$1.00 \times 10^{12}$ | [*]$2.54 \times 10^{10}$ |
| | 0 | | $2.60 \times 10^{10}$ |
| | 2 | | $2.53 \times 10^{10}$ |
| | 4 | | $2.49 \times 10^{10}$ |
| | 8 | | $2.59 \times 10^{10}$ |
| [*]value normalized with 2.5 dilution | | | |

Data from Table 3 demonstrate that there was not any significant loss of infective units during liquid processing of the formulation. Therefore, using as buffer the mixture of Tris and glycine not only allows avoiding toxic glycerol in the composition but also provides a suitable environment for liquid and for lyophilized adenovirus containing compositions.

2. Analysis of the impact of pH on stability of adenovirus particles: Aggregation and Functionality of adenovirus particles.

Next assay depicts the effect of the chosen buffer system on the adenovirus-containing compositions. In order to determine the effect of using a buffer system with a protonic acid (HCl) in comparison with an aprotic buffer, several compositions were tested. A concentration of $1 \times 10^{12}$ particles of Ad5/ml (pv/mL) was used in each tested liquid stock, thus resembling a liquid processing before lyophylization.

Z-average, measured by Dynamic Light Scattering (DLS), and the corresponding polydispersity index (PDI) are indicated below. These parameters relate with the size of aggregates of particles (Z-average in nm) and with size distribution of particles (PDI). For a perfectly uniform sample, the PDI would be 0.0. PDI from 0.0 to 0.1 reflects a narrow non-uniform distribution type sample. From 0.1 to 0.4 the PDI indicates a moderate non-uniform distribution type sample. PDI higher than 0.4 is usually understood as a broad distribution type sample.

Data were taken using the Malvern Zetasizer Nano ZS (ZEN3600) with a detection angle of 173° and a red laser of 632 nm at 25 °C. For all analysis a polystyrene standard (100 nm) was used (Thermo Scientific). Five determinations (measures) were taken from a 200 µl of sample in polystyrene cubes (Starstedt). Z-average and PDI is the mean value of these five determinations.

Table 4. Aggregation

| Time (h) | Tris 10 mM- HCl pH 8.0, MgCl$_2$ 1mM, NaCl 75 mM, 7% (p/v) trehalose, 2% (p/v) mannitol, 1% (p/v) saccharose (Stock B1) | | Tris 25 mM - Glycine 0.192 M pH 8.5, MgCl$_2$ 1mM, NaCl 75 mM, 7% (p/v) trehalose, 2% (p/v) mannitol, 1% (p/v) saccharose (Stock D) | | Tris 25 mM - Glycine 0.192 M pH 8.5, MgCl$_2$ 1mM, NaCl 75 mM, 7% (p/v) trehalose, 2% (p/v) mannitol, 1% (p/v) saccharose, albumin 0.4% (p/v) (Stock E) (sample LP0243_81 From Table 2) | |
|---|---|---|---|---|---|---|
| | Z-average (nm) | PDI | Z-average (nm) | PDI | Z-average (nm) | PDI |
| T(0) | 3725 | 0.20 | 154 | 0.07 | 145 | 0.04 |
| 1 | 2673 | 0.28 | 155 | 0.09 | 146 | 0.06 |
| 2 | 3802 | 0.20 | 157 | 0.16 | 147 | 0.05 |
| 3 | | | 165 | 0.16 | 147 | 0.06 |
| 4 | | | 263 | 0.31 | 150 | 0.07 |
| 5 | | | 570 | 0.54 | 151 | 0.06 |

As can be deduced from table 4, stocks D and E have a proper particle size at t(0), since a size from 145-154 nm is equivalent to a hydrodynamic diameter of Ad5 (about 117 nm) and considering multiple excipients interacting with the particles. At this t(0) PDI are low, which means that the compositions assure stability of the particles. Stock E maintains size and polydispersity along time, while in stock D only a slight increase on aggregation is observed when the compositions are in solution. In contrast, the adenovirus-containing composition with a buffer including the acid HCl (a buffer with an protonic acid) supposed bigger aggregates than those in the compositions comprising the aprotic buffer system (Tris and glycine).

As depicted in Table 2, a composition as that of stock E (or sample LP0243_81) implied good functionality values (IU/ml) even after being stored in lyophilised form from 3 and 6 months at -20°C.

Therefore, with the proposed buffer consisting in a mixture of Tris and glycine high initial amounts of viral particles ($1 \times 10^{12}$ pv/ml) could be employed without the drawback of loss for aggregation during processing in liquid form and during lyophilisation. Thus, functionality of viral particles was preserved. In addition, the adenovirus containing compositions, even being free of glycerol, assure stability of the viral particles for a long time.

It was also analysed in a parallel assay, the pH effect in adenovirus containing compositions with a buffer system comprising Tris-HCl. This was done in an attempt to simulate the conditions that take place during lyophilisation meanwhile the sample is getting frozen and in the presence of protons. Data in next Table 5 demonstrate that lowering of the pH (due to the presence of protons provided by the buffer) from 7.4 to 7.2 in the buffer system comprising Tris-HCl imply an increase of aggregation of the viral particles. On the other hand, basification (to pH = 8.9) tends to reduce particle size.

Table 5. Effect of protons provided by protonic buffer systems

| Tris - HCl 10 mM pH 7.4 , NaCl 75 mM, MgCl$_2$ 1mM i 5% (p/v) trehalose | | |
|---|---|---|
| pH | Z-average (nm) | pdl |
| 7.3 | 2011 | 0.42 |
| 7.2 | 3965 | 0.33 |
| 8.9 | 800 | 0.69 |

Thus, the buffer system comprising glycine and Tris, as the only buffer components, in an adenovirus containing composition is a good and advantageous choice for preserving adenovirus particles.

**Citation List**

Patent Literature

- US20080102508
- US7456009

Non Patent Literature

- Altaras et al., "Production and Formulation of Adenovirus Vectors", Adv. Biochem. Engin/Biotechnol-2005, vol. no. 99, pp.: 193-260

## Claims

1. A pharmaceutical adenovirus containing composition, which is free of glycerol, comprising:

   - a therapeutically effective amount of one or more adenovirus types;
   - a buffer system comprising tris(hydroxymethyl) aminomethane and glycine;
   - a cryoprotectant mixture comprising (a) trehalose, (b) mannitol, and (c) saccharose;
   - albumin; and
   - a mixture of inorganic salts comprising sodium chloride and magnesium chloride.

2. The pharmaceutical composition according to claim 1, further comprising additional pharmaceutically acceptable excipients and/or carriers.

3. The pharmaceutical composition according to any one of claims 1-2, wherein the tris(hydroxymethyl) aminomethane and glycine are the only buffer components in the composition.

4. The pharmaceutical composition according to any of one of claims 1-3, which is a liquid aqueous composition at a temperature from 14 °C to 35 °C.

5. The pharmaceutical composition according to claim 4, which has a pH from 7 to 9, and wherein the concentration of tris(hydroxymethyl) aminomethane is from 5 to 30 mM and the concentration of glycine is from 0.15 M to 0.25 M.

6. The pharmaceutical composition according to any of one of claims 4-5, wherein the weight/volume percentage of trehalose is from 7 % to 15 %; the weight/volume percentage of mannitol is from 2 % to 3 %; the weight/volume percentage of saccharose is from 1 % to 2 %; and the weight/volume percentage of albumin is from 0.04 % to 0.5 %; being the weight/volume percentage of any compound defined by the amount in grams of the compound in a 100 ml of the liquid aqueous composition.

7. The pharmaceutical composition according to any of one of claims 1-3, which is a lyophilised composition.

8. The pharmaceutical composition according to any one of claims 1- 7, wherein the one or more adenovirus type is selected from the group consisting of one or more human adenovirus of C serotype, one or more human adenovirus of D serotype, and combinations thereof.

9. The pharmaceutical composition according to claim 8, wherein the one or more adenovirus type is a human adenovirus of C serotype selected from the group consisting of Ad1, Ad2, Ad5, Ad6, and combinations thereof.

10. A method for preparing a pharmaceutical adenovirus containing composition as defined in any of claims 1-9, the method comprising the following steps:

   (a) preparing a solution in water by adding a cryoprotectant mixture comprising trehalose, mannitol, saccharose; a buffer system comprising tris(hydroxymethyl) aminomethane and glycine; and a mixture of inorganic salts comprising sodium chloride and magnesium chloride;
   (b) sterilization filtration of the solution obtained in step (a);
   (c) adding albumin under sterile conditions;
   (d) maintaining the solution of step (c) under refrigeration at a temperature from 2°C to 10 °C and under agitation for the necessary period of time to obtain an homogenized solution comprising the buffer system, the inorganic salts and the cryoprotectant mixture; and

(e) mixing the homogenized solution of step (d) with an effective amount of adenovirus to obtain a liquid pharmaceutical adenovirus containing composition.

11. The method according to claim 10, which further comprises a step of lyophilisation.

12. The method according to claim 11, wherein the lyophilisation is carried out by:

- lowering the liquid pharmaceutical adenovirus containing composition to a temperature from -60°C to -50 °C;
- a first drying step at a temperature from -45 °C to -30 °C at a pressure from 5.0 Pa to 8.0 Pa; and
- a second drying step in which the temperature is elevated to a temperature from 10 °C to 40 °C, and which temperature is maintained for a necessary period of time to obtain a lyophilized cake with a residual humidity of from 1.0 % to 3.0 % by weight in the final lyophilised composition.

13. A pharmaceutical adenovirus containing composition as defined in any one of claims 1-9 for use as a vaccine.

14. A buffered composition suitable for stabilizing adenovirus that have to be stored in lyophilized form, said composition being free of glycerol and comprising:

- a buffer system comprising tris(hydroxymethyl) aminomethane (Tris) and glycine;
- a cryoprotectant mixture comprising (a) trehalose, (b) mannitol, and (c) saccharose; - albumin; and
- a mixture of inorganic salts comprising sodium chloride and magnesium chloride.

15. Use of a buffered composition comprising:

- a buffer system comprising tris(hydroxymethyl) aminomethane (Tris) and glycine;
- a cryoprotectant mixture comprising (a) trehalose, (b) mannitol, and (c) saccharose;
- albumin; and
- a mixture of inorganic salts comprising sodium chloride and magnesium chloride; as an adenovirus stabilizing composition.

**Patentansprüche**

1. Eine pharmazeutische Adenovirus enthaltende Zusammensetzung, welche frei von Glycerin ist, umfassend:

- eine therapeutisch wirksame Menge von einem oder mehreren Adenovirus-Typen;
- ein Puffersystem umfassend Tris(hydroxymethyl)aminomethan und Glycin;
- ein Kälteschutzmittelgemisch umfassend (a) Trehalose, (b) Mannitol und (c) Saccharose;
- Albumin; und
- eine Mischung aus anorganischen Salzen, die Natriumchlorid und Magnesiumchlorid enthält.

2. Die pharmazeutische Zusammensetzung nach Anspruch 1, weiterhin umfassend zusätzliche pharmazeutisch akzeptable Hilfsstoffe und/oder Trägersubstanzen.

3. Die pharmazeutische Zusammensetzung nach einem der Ansprüche 1 bis 2, wobei das Tris(hydroxymethyl)aminomethan und das Glycin die einzigen Pufferkomponenten in der Zusammensetzung sind.

4. Die pharmazeutische Zusammensetzung nach einem der Ansprüche 1 bis 3, welche eine flüssige wässrige Zusammensetzung bei einer Temperatur von 14 °C bis 35 °C ist.

5. Die pharmazeutische Zusammensetzung nach Anspruch 4, welche einen pH-Wert von 7 bis 9 hat und wobei die Konzentration von Tris(hydroxymethyl)aminomethan von 5 bis 30 mM ist und die Konzentration von Glycin von 0,15 M bis 0,25 M ist.

6. Die pharmazeutische Zusammensetzung nach einem der Ansprüche 4 bis 5, wobei der Gewichts-/Volumenprozentsatz der Trehalose von 7 % bis 15 % ist; der Gewichts-/Volumenprozentsatz des Mannitols von 2 % bis 3 % ist; der Gewichts-/Volumenprozentsatz der Saccharose von 1 % bis 2 % ist; und der Gewichts-/Volumenprozentsatz des Albumins von 0,04 % bis 0,5 % ist; wobei der Gewichts-/Volumenprozentsatz einer beliebigen Verbindung durch die Menge in Gramm der Verbindung in 100 ml der flüssigen wässrigen Zusammensetzung definiert ist.

7. Die pharmazeutische Zusammensetzung nach einem der Ansprüche 1 bis 3, welche eine lyophilisierte Zusammensetzung ist.

8. Die pharmazeutische Zusammensetzung nach einem der Ansprüche 1 bis 7, wobei der eine oder die mehreren Adenovirus-Typen aus der Gruppe ausgewählt ist, die aus einem oder mehreren humanen Adenoviren des C-Serotyps, einem oder mehreren humanen Adenoviren des D-Serotyps und Kombinationen davon besteht.

9. Die pharmazeutische Zusammensetzung nach Anspruch 8, wobei der eine oder die mehreren Adenovirus-Typen ein menschliches Adenovirus des C-Serotyps ist, ausgewählt aus der Gruppe bestehend aus Ad1, Ad2, Ad5, Ad6 und Kombinationen davon.

10. Ein Verfahren zur Herstellung einer pharmazeutischen Adenovirus enthaltenden Zusammensetzung wie in einem der Ansprüche 1-9 definiert, wobei das Verfahren die folgenden Schritte umfasst:

(a) Herstellen einer Lösung in Wasser durch Zugabe eines Kälteschutzmittelgemisches umfassend Trehalose, Mannitol, Saccharose; eines Puffersystems umfassend Tris(hydroxymethyl)aminomethan und Glycin; und einer Mischung aus anorganischen Salzen umfassend Natriumchlorid und Magnesiumchlorid;
(b) Sterilisationsfiltration der in Schritt (a) erhaltenen Lösung;
(c) Zugabe von Albumin unter sterilen Bedingungen;
(d) Halten der Lösung aus Schritt (c) unter Kühlung bei einer Temperatur von 2 °C bis 10 °C und unter Rühren für den erforderlichen Zeitraum, um eine homogenisierte Lösung zu erhalten, die das Puffersystem, die anorganischen Salze und das Kälteschutzmittelgemisch umfasst; und
(e) Mischen der homogenisierten Lösung von Schritt (d) mit einer wirksamen Menge an Adenovirus, um eine flüssige pharmazeutische Adenovirus enthaltende Zusammensetzung zu erhalten.

11. Das Verfahren nach Anspruch 10, welches weiterhin einen Schritt der Gefriertrocknung umfasst.

12. Das Verfahren nach Anspruch 11, wobei die Gefriertrocknung durchgeführt wird durch:

- Absenken der flüssigen pharmazeutischen Adenovirus enthaltenden Zusammensetzung auf eine Temperatur von -60 °C bis -50 °C;
- einen ersten Trocknungsschritt bei einer Temperatur von -45 °C bis -30 °C bei einem Druck von 5,0 Pa bis 8,0 Pa; und
- einen zweiten Trocknungsschritt, bei dem die Temperatur auf eine Temperatur von 10 °C bis 40 °C erhöht wird und die Temperatur für einen erforderlichen Zeitraum aufrechterhalten wird, um einen lyophilisierten Kuchen mit einer Restfeuchtigkeit von 1,0 bis 3,0 Gew.- % in der endgültigen lyophilisierten Zusammensetzung zu erhalten.

13. Eine pharmazeutische Adenovirus enthaltende Zusammensetzung wie in einem der Ansprüche 1 bis 9 definiert zur Verwendung als Impfstoff.

14. Eine gepufferte Zusammensetzung, die zur Stabilisierung von Adenoviren geeignet ist, die in lyophilisierter Form gelagert werden müssen, wobei die Zusammensetzung frei von Glycerin ist und Folgendes umfasst:

- ein Puffersystem umfassend Tris(hydroxymethyl)aminomethan (Tris) und Glycin;
- ein Kälteschutzmittelgemisch umfassend (a) Trehalose, (b) Mannitol und (c) Saccharose; - Albumin; und
- eine Mischung aus anorganischen Salzen, die Natriumchlorid und Magnesiumchlorid enthält.

15. Verwendung von einer gepufferten Zusammensetzung umfassend:

- ein Puffersystem umfassend Tris(hydroxymethyl)aminomethan (Tris) und Glycin;
- ein Kälteschutzmittelgemisch umfassend (a) Trehalose, (b) Mannitol und (c) Saccharose;
- Albumin; und
- eine Mischung aus anorganischen Salzen, die Natriumchlorid und Magnesiumchlorid umfasst; als eine Adenovirus stabilisierende Zusammensetzung.

**Revendications**

1. Une composition pharmaceutique contenant un adénovirus, qui est exempte de glycérol, comprenant :

   - une quantité thérapeutiquement efficace d'un ou de plusieurs types d'adénovirus ;
   - un système tampon comprenant du tris(hydroxyméthyl) aminométhane et de la glycine ;
   - un mélange cryoprotecteur comprenant (a) du tréhalose, (b) du mannitol et (c) du saccharose ;
   - de l'albumine ; et
   - un mélange de sels inorganiques comprenant du chlorure de sodium et du chlorure de magnésium.

2. La composition pharmaceutique selon la revendication 1, comprenant en outre des excipients et/ou des véhicules pharmaceutiquement acceptables supplémentaires.

3. La composition pharmaceutique selon l'une quelconque des revendications 1 à 2, dans laquelle le tris(hydroxyméthyl) aminométhane et la glycine sont les seuls composants du tampon de la composition.

4. La composition pharmaceutique selon l'une quelconque des revendications 1 à 3, qui est une composition aqueuse liquide à une température de 14 °C à 35 °C.

5. La composition pharmaceutique selon la revendication 4, qui a un pH de 7 à 9, et dans laquelle la concentration de tris(hydroxyméthyl) aminométhane est de 5 à 30 mM et la concentration de la glycine est de 0,15 M à 0,25 M.

6. La composition pharmaceutique selon l'une quelconque des revendications 4 à 5, dans laquelle le pourcentage en poids/volume de tréhalose est de 7 % à 15 % ; le pourcentage en poids/volume de mannitol est de 2 % à 3 % ; le pourcentage en poids/volume de saccharose est de 1 % à 2 % ; et le pourcentage en poids/volume d'albumine est de 0,04 % à 0,5 % ; étant le pourcentage en poids/volume de tout composé défini par la quantité en grammes du composé dans 100 ml de la composition aqueuse liquide.

7. La composition pharmaceutique selon l'une quelconque des revendications 1 à 3, qui est une composition lyophilisée.

8. La composition pharmaceutique selon l'une quelconque des revendications 1 à 7, dans laquelle le ou les types d'adénovirus sont choisis dans le groupe constitué par un ou plusieurs adénovirus humains de sérotype C, un ou plusieurs adénovirus humains de sérotype D et des combinaisons de ceux-ci.

9. La composition pharmaceutique selon la revendication 8, dans laquelle le ou les types d'adénovirus est un adénovirus humain de sérotype C choisi dans le groupe constitué de Ad1, Ad2, Ad5, Ad6 et des combinaisons de ceux-ci.

10. Un procédé de préparation d'une composition pharmaceutique contenant un adénovirus telle que définie dans l'une quelconque des revendications 1 à 9, le procédé comprenant les étapes suivantes :

    (a) préparer une solution dans l'eau en ajoutant un mélange cryoprotecteur comprenant du tréhalose, du mannitol, du saccharose ; un système tampon comprenant du tris(hydroxyméthyl) aminométhane et de la glycine ; et un mélange de sels inorganiques comprenant du chlorure de sodium et du chlorure de magnésium ;
    (b) filtration de stérilisation de la solution obtenue à l'étape (a) ;
    (c) ajouter de l'albumine dans des conditions stériles ;
    (d) maintenir la solution de l'étape (c) sous réfrigération à une température de 2 °C à 10 °C et sous agitation pendant la période de temps nécessaire pour obtenir une solution homogénéisée comprenant le système tampon, les sels inorganiques et le mélange cryoprotecteur ; et
    (e) mélanger la solution homogénéisée de l'étape (d) avec une quantité efficace d'adénovirus pour obtenir une composition pharmaceutique liquide contenant un adénovirus.

11. Le procédé selon la revendication 10, qui comprend en outre une étape de lyophilisation.

12. Le procédé selon la revendication 11, dans lequel la lyophilisation est réalisée comme suit :

    - abaisser la composition pharmaceutique liquide contenant l'adénovirus à une température de -60 °C à -50 °C ;
    - une première étape de séchage à une température de -45 °C à -30 °C à une pression de 5,0 Pa à 8,0 Pa ; et
    - une seconde étape de séchage dans laquelle la température est élevée à une température de 10 °C à 40 °C, et laquelle température est maintenue pendant une période de temps nécessaire pour obtenir un gâteau lyophilisé avec une humidité résiduelle de 1,0 % à 3,0 % en poids dans la composition lyophilisée finale.

13. Une composition pharmaceutique contenant un adénovirus telle que définie dans l'une quelconque des revendications 1 à 9 pour une utilisation en tant que vaccin.

14. Une composition tamponnée adaptée pour stabiliser des adénovirus devant être conservés sous forme lyophilisée, ladite composition étant exempte de glycérol et comprenant :

   - un système tampon comprenant du tris(hydroxyméthyl) aminométhane (Tris) et de la glycine ;
   - un mélange cryoprotecteur comprenant (a) du tréhalose, (b) du mannitol et (c) du saccharose ; - de l'albumine ; et
   - un mélange de sels inorganiques comprenant du chlorure de sodium et du chlorure de magnésium.

15. Utilisation d'une composition tamponnée comprenant :

   - un système tampon comprenant du tris(hydroxyméthyl) aminométhane (Tris) et de la glycine ;
   - un mélange cryoprotecteur comprenant (a) du tréhalose, (b) du mannitol et (c) du saccharose ;
   - de l'albumine ; et
   - un mélange de sels inorganiques comprenant du chlorure de sodium et du chlorure de magnésium ; comme composition stabilisante des adénovirus.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20080102508 A

- US 7456009 B

**Non-patent literature cited in the description**

- **ALTARAS et al.** Production and Formulation of Adenovirus Vectors. *Adv. Biochem. Engin/Biotechnol,* 2005, (99), 193-260

- **ALTARAS et al.** Production and Formulation of Adenovirus Vectors. *Adv. Biochem. Engin/Biotechnol,* 2005, vol. 99, 193-260